# EUROPÄISCHE PATENTSCHRIFT

(11) **EP 2 779 932 B1**
(45) Veröffentlichungstag und Bekanntmachung des Hinweises auf die Patenterteilung: **21.08.2019**
(21) Anmeldenummer: 12797779.1
(22) Anmeldetag: 15.11.2012
(51) Int. Cl.: A61B 34/20

(54) **REGISTRIERVERFAHREN, POSITIONSERFASSUNGSSYSTEM UND ABTASTINSTRUMENT**
REGISTERING METHOD, POSITION DETECTING SYSTEM, AND SCANNING INSTRUMENT
PROCÉDÉ D'ENREGISTREMENT, SYSTÈME DE DÉTECTION DE POSITION ET INSTRUMENT PALPEUR

(30) Priorität: 15.11.2011 DE 102011119073
(43) Veröffentlichungstag der Anmeldung: 24.09.2014
(73) Patentinhaber: Fiagon AG Medical Technologies, 16761 Hennigsdorf (DE)
(72) Erfinder: KRUEGER, Timo, 13465 Berlin (DE); MUCHA, Dirk, 13467 Berlin (DE)
(74) Vertreter: Eisenführ Speiser
(86) Internationale Anmeldenummer: PCT/EP2012/072783
(87) Internationale Veröffentlichungsnummer: WO 2013/072434

(56) Entgegenhaltungen:
- EP-A2- 1 523 951
- US-A- 6 011 987
- US-A1- 2005 101 966
- US-A1- 2008 319 448

## Beschreibung

Die Erfindung betrifft ein Verfahren zum Zuordnen von mittels eines Positionserfassungssystems erfassten Positionswerten zu einem topographischen Abbild eines Objektes oder Körperteils. Außerdem betrifft die Erfindung ein Positionserfassungssystem mit einem beweglichen Abtastinstrument sowie ein Abtastinstrument selbst.

Positionserfassungssysteme, die beispielsweise im medizinischen Bereich eine Navigation von Instrumenten, beispielsweise chirurgischer Instrumente, unterstützen, sind grundsätzlich bekannt. Solche Positionserfassungssysteme können optische, Ultraschallgestützte oder elektromagnetische Positionserfassungssysteme sein. So sind beispielsweise elektromagnetische Positionserfassungssysteme bekannt, bei denen ein Feldgenerator ein elektromagnetisches Wechselfeld erzeugt und Positionssensoren vorgesehen sind, die Spulen aufweisen. Durch das elektromagnetische Wechselfeld des Generators werden in den Spulen Ströme induziert, die von der Ausrichtung einer jeweiligen Spule zum elektromagnetischen Wechselfeld abhängen. Wenn ein bewegliches Instrument mit derartigen Positionssensoren in Form von Sensorspulen ausgerichtet ist, ist es möglich, Ort und Lage des Instruments relativ zu einem Referenzsensor, der beispielsweise ebenfalls Spulen aufweisen kann, zu bestimmen. Vorzugsweise ist hierbei der Referenzsensor als Patientenlokalisator fest mit einem Körperteil eines Patienten (oder auch einem anderen Objekt) verbunden.

Für eine Navigation in Körperteilen eines Patienten wird typischerweise die Position eines Instrumentes mit einem derartigen Positionserfassungssystem erfasst und die Position des Instrumentes in tomographisch gewonnenen Schnittbildern des Körperteils angezeigt. Damit dies funktioniert, müssen die Positionswerte, die der Positionssensor des Instrumentes liefert, in Koordinaten des tomographischen Abbildes des Patienten übertragen werden. Dazu ist es beispielsweise bekannt, aus einem tomographischen Abbild eines Patienten ein topographisches Abbild der Oberfläche eines Körperteils zu generieren, um Punkte auf der Oberfläche des topographischen Abbilds (im Folgenden auch Modelloberfläche genannt) solchen Punkten auf der Oberfläche des realen Körperteils zuzuordnen, die jeweils von einem Zeige- oder Abtastinstrument berührt werden, kann im Rahmen eines Registrierverfahrens eine Transformationsvorschrift für mittels des Positionserfassungssystems erfasste Positionswerte in Modellkoordinaten erstellt werden. Dazu werden auf der realen Oberfläche des Körperteils eine Mehrzahl von Punkten abgetastet und die zugehörigen Positionswerte, die ja Punkte auf einer realen Oberfläche repräsentieren, werden Punkten auf der Modelloberfläche unter Beibehaltung ihrer relativen Position zueinander so zugeordnet, dass sich ein möglichst kleiner Fehler ergibt. Hieraus ergibt sich eine Transformationsvorschrift, die angibt, wie erfasste Positionswerte in Koordinaten des topographischen Abbildes - hier auch als topographisches Modell bezeichnet - und damit auch in Koordinaten des tomographischen Abbildes oder Modells, umzurechnen sind.

US 2005/101966 A1 offenbart ein Positionserfassungssystem mit einem beweglichen Abtastinstrument, wobei das bewegliche Abtastinstrument eine Abtastspitze mit einer teilkugelförmigen Außenoberfläche aufweist und der Kugelmittelpunkt der sphärischen Abtastspitze als Positionsreferenzpunkt dient und das Positionserfassungssystem weiterhin einen Speicher für ein topographisches Modell eines Objektes oder Körperteils aufweist.

Der Erfindung liegt die Aufgabe zugrunde, ein solches an sich bekanntes Verfahren, Positionserfassungssystem und Abtastinstrument weiterzuentwickeln.

Erfindungsgemäß wird diese Aufgabe zum einen durch ein Verfahren der eingangs genannten Art gelöst, welches die folgenden Schritte umfasst:
Bereitstellen eines topographischen Modells eines Objekts oder Körperteils
Abtasten einer Oberfläche des Objekts oder Körperteils
Erfassen von Positionswerten während des Abtastens und
Zuordnen erfasster Positionswerte zu entsprechenden Positionen in dem topographischen Modell, wobei die erfassten Positionswerte Punkten einer virtuellen Oberfläche des topographischen Modells zugeordnet werden, die von einer Modelloberfläche einen Abstand hat, der einen Radius einer teilkugelförmigen Abtastspitze eines Abtastinstrumentes entspricht, mit dem die Oberfläche des Objekts oder Körperteils jeweils abgetastet wird.

Vorzugsweise wird als Positionserfassungssystem ein Positionserfassungssystem mit einem beweglichen Abtastinstrument verwendet wird, welches eine Abtastspitze mit einer sphärischen Außenoberfläche und mit einem Kugelmittelpunkt als Positionsreferenzpunkt aufweist. Für das Zuordnen mittels des Abtastinstruments erfasster Positionswerte zu einer Punkten wird ausgehend von einer durch das topografische Modell definierten Modelloberfläche eine virtuelle Modelloberfläche bestimmt, die von der Modelloberfläche einen Abstand hat, der gleich dem Abstand des Kugelmittelpunktes der Abtastspitze von deren sphärischer Außenoberfläche ist. Mit einem Beweglichen Abtastinstrument ist ein Instrument geeint, dass relativ zum abzutastenden Körperteil oder Objekt beweglich ist, im Unterschied beispielsweise zu einem objekt- oder körperteilfesten Positionssensor.

Ein derartiges Verfahren erlaubt es, Abtastinstrumente einzusetzen, deren Abtastspitze nicht im eigentlichen Sinne spitz ist, sondern vielmehr eine sphärische Oberfläche aufweist, d. h. eine Oberfläche, die einer Teilkugel entspricht. Der Durchmesser der teilkugelförmigen Abtastspitze kann dabei vergleichsweise groß gewählt werden, beispielsweise größer als ein die Abtastspitze mit einem Handgriff verbindender Schaft des Abtastinstruments. Ein derartiges Abtastinstrument mit einer Abtastspitze mit relativ großem Kugelradius kann leichter und sanfter über eine weiche Oberfläche geführt werden, wie sie beispielsweise in Teilen des Gesichts eines Patienten vorzufinden ist.

Nachteil eines derartigen Abtastinstruments mit einer kugelförmigen Abtastspitze ist es, dass das Positionserfassungssystem nicht erfassen kann, mit welchem Punkt der Kugeloberfläche das Abtastinstrument gerade die jeweilige Oberfläche berührt. Beispielsweise kann dieser Punkt in genauer Verlängerung einer Längsachse eines die Abtastspitze mit einem Handgriff verbindenden Schafts liegen, wenn das Abtastinstrument genau senkrecht auf die Oberfläche aufgesetzt wird. Wird jedoch das Abtastinstrument schräg auf die Oberfläche aufgesetzt, berührt ein anderer Punkt der kugelförmigen Abtastspitze die Oberfläche des Körperteils, und es ergäbe sich ein Positionsfehler, der leicht wenige Millimeter betragen kann und von daher nicht reparierbar ist. Um diesen Fehler zu kompensieren, werden erfindungsgemäß aus den Oberflächen des tomographischen Modells oder topographischen Modells virtuelle Oberflächen errechnet, die von den eigentlichen Modelloberflächen einen Abstand haben, der gerade dem Radius der teilkugelförmigen Abtastspitze des Abtastinstrumentes entspricht. Dann kann als Positionsreferenzpunkt des Abtastinstrumentes der Mittelpunkt der teilkugelförmigen Spitze des Abtastinstrumentes verwendet werden, dessen relative Position sowohl zur Modelloberfläche als auch zur virtuellen Modelloberfläche unabhängig von dem Winkel ist, in dem das Instrument auf die jeweilige Oberfläche des Körperteils oder Objekts aufgesetzt wird.

Im Rahmen eines erfindungsgemäßen Registrierverfahrens werden erfasste Positionswerte, die auf den Kugelmittelpunkt der teilkugelförmigen Abtastspitze als Positionsreferenzpunkt bezogen sind, Positionen auf der virtuellen Modelloberfläche derart zugeordnet, dass unter Beibehaltung der relativen Position der erfassten Positionswerte zueinander und Anwendung einer einheitlichen Transformationsvorschrift für alle Positionswerte eine möglichst passgenaue Übereinstimmung der erfassten Positionswerte mit Positionen auf der virtuellen Modelloberfläche ergibt.

Erfindungsgemäß wird die Aufgabe auch durch ein Positionserfassungssystem mit einem beweglichen Abtastinstrument gelöst, wobei das bewegliche Abtastinstrument eine Abtastspitze mit einer sphärischen, also teilkugelförmigen Außenoberfläche aufweist, wobei der Kugelmittelpunkt der sphärischen Abtastspitze als Positionsreferenzpunkt dient. Das Positionserfassungssystem weist weiterhin einen Speicher für ein topographisches Modell eines Objektes oder Körperteils auf und ist erfindungsgemäß dazu ausgebildet, von einem beweglichen Instrument erfasste Positionswerte mittels einer Transformationsvorschrift in ein dem topographischen Modell zugrundeliegendes Modellkoordinatensystem zu übertragen. Das Positionserfassungssystem ist weiter dazu ausgebildet, ein Zuordnen mittels des Abtastinstruments mit teilkugelförmiger Abtastspitze erfasster Positionswerte zu Punkten oder Positionen auf einer durch das topographische Modell definierten Modelloberfläche durchzuführen, indem das Positionserfassungssystem auf den Kugelmittelpunkt der Abtastspitze als Positionsreferenzpunkt bezogene Positionswerte einer virtuellen Oberfläche zuordnet, die von einer durch das topographische Modell definierten Modelloberfläche einen Abstand hat, der genau dem Kugelradius der teilkugelförmigen Abtastspitze entspricht.

Zum Zwecke der Registrierung kann das Positionserfassungssystem zusätzlich dazu ausgebildet sein, eine Transformationsvorschrift zum Umrechnen erfasster Positionswerte in Modellkoordinaten zu ermitteln, wobei die Transformationsvorschrift die Eigenschaft hat, bei der Zuordnung im Rahmen einer Registrierung erfasste Positionswerte zu Punkten bzw. Positionen auf der virtuellen Modelloberfläche eine möglichst geringe Gesamtabweichung erfasster Positionswerte von zugeordneten Punkten auf der virtuellen Modelloberfläche zu ergeben.

Erfindungsgemäß wird die zugrundeliegende Aufgabe auch durch ein Abtastinstrument gelöst, welches eine teilkugelförmige Abtastspitze aufweist, die vorzugsweise einen Durchmesser von mehr als 3 mm besitzt. Gemäß einer bevorzugten Ausführungsvariante kann die teilkugelförmige Abtastspitze von einer Kugel gebildet sein, die nach Art einer Kugelschreiberspitze drehbar ist. Auch kann die Abtastspitze ausgebildet sein, ein Gleitgel oder ähnliches abzugeben, so dass die Abtastspitze leichter über eine abzutastende Oberfläche geiten kann.

Die Erfindung soll nun anhand eines Ausführungsbeispiels mit Bezug auf die Figuren näher erläutert werden. Von diesen zeigt:
- Fig. 1: ein erfindungsgemäßes Positionserfassungssystem mit einem Feldgenerator für ein elektromagnetisches Wechselfeld, einem patientenfesten Positionssensor (Patientenlokalisator) und einem beweglichen Abtastinstrument;
- Fig. 2: eine Skizze eines distalen Endes eines erfindungsgemäßen Abtastinstrumentes mit teilkugelförmiger Abtastspitze und einer angedeuteten virtuellen Oberfläche zur Erläuterung des der Erfindung zugrundeliegenden Prinzips.

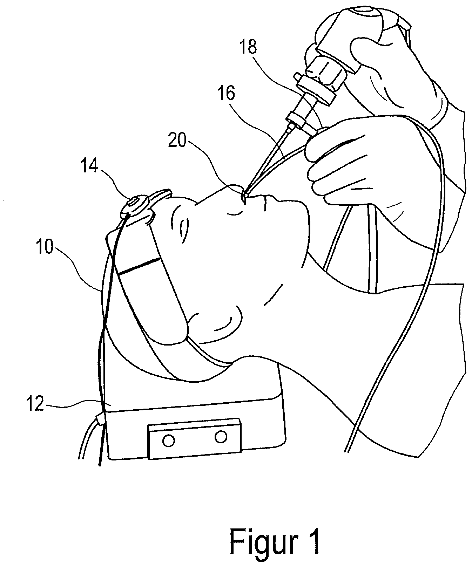

Fig. 1 zeigt skizzenhaft einen Kopf 10 eines Patienten in einer Seitenansicht. In der Nachbarschaft des Kopfes 10 befindet sich ein Feldgenerator 12 zum Erzeugen eines elektromagnetischen Wechselfeldes, das im Bereich des Kopfes des Patienten zu erfassen ist. Am Kopf 10 des Patienten ist ein patientenfester Positionssensor als Referenzpositionssensor 14 bzw. Patientenlokalisator befestigt. Ein bewegliches Abtastinstrument 16 besitzt ein proximales Ende mit einem Handgriff 18 und ein distales Ende mit einer teilkugelförmigen Abtastspitze 20. An dem oder in dem Abtastinstrument 16 ist ein Instrumentenpositionssensor 22 befestigt. Der Referenzpositionssensor 14 und der Instrumentenpositionssensor 22 weisen jeweils eine oder mehrere elektrische Spulen auf. Das von dem Feldgenerator 12 im Betrieb ausgehende elektromagnetische Wechselfeld induziert in den Spulen jeweils einen Strom, dessen Amplitude (und Phase?) von der relativen Position und Ausrichtung einer jeweiligen Sensorspule zum Feldgenerator 12 abhängt. Auf diese Weise ist es in an sich bekannter Weise möglich, die relative Position und Ausrichtung des Instrumentenpositionssensors in Bezug auf den Referenzpositionssensor zu bestimmen.

Um mittels eines Positionserfassungssystems erfasste Positionswerte eines Instrumentes für eine Instrumentennavigation in dem Sinne zu nutzen, dass eine jeweils aktuelle Position eines Instrumentes in beispielsweise tomographisch gewonnenen Schnittbildern eines Körperteils angezeigt wird, müssen die vom Positionssensor des Instrumentes gelieferten Positionswerte in ein einem tomographischen Abbild zugrundeliegendes Modellkoordinatensystem transformiert werden. Eine hierzu nötige Transformationsvorschrift wird in an sich bekannter Weise mittels eines Registrierverfahrens gewonnen. Dazu wird beispielsweise mit einem Zeige- oder Abtastinstrument eine Oberfläche eines Körperteils abgetastet, und dabei gewonnene Positionswerte werden mit möglichst kleinem Fehler auf einer von einem tomographischen oder topographischen Abbild abgeleitete Modelloberfläche übertragen. Die für diese möglichst genaue Übertragung erforderlichen Skalierungen, Rotationen und/oder Translationen oder dergleichen ergeben schließlich die Transformationsvorschrift.

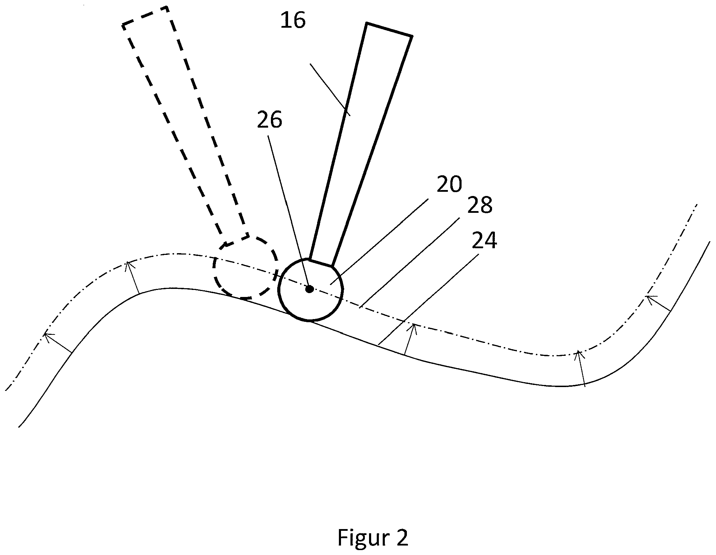

Fig. 2 zeigt ein distales Ende eines Abtastinstrumentes 16 mit einer teilkugelförmigen Abtastspitze 20. Das distale Ende des Abtastinstrumentes 16 ist in Fig. 2 zweimal dargestellt, nämlich einmal in durchgezogener Linie und einmal gestrichelt. Mittels der gestrichelten Darstellung ist angedeutet, dass ein anderer Punkt der teilkugelförmigen Abtastspitze die Oberfläche 24 eines Objekts oder Körperteils berührt, wenn das Abtastinstrument 16 in einen anderen Winkel auf diese Oberfläche 24 aufgesetzt wird. Da der Winkel, mit dem ein Abtastinstrument auf die jeweilige Oberfläche aufgesetzt wird, in der Regel nicht bekannt ist, ist mit einem Abtastinstrument mit teilkugelförmiger Abtastspitze keine genaue Positionsbestimmung auf Basis des Berührungspunkts als Referenzpunkt möglich. Erfindungsgemäß wird daher der Kugelmittelpunkt 26 der teilkugelförmigen Abtastspitze 20 als Positionsreferenzpunkt des Abtastinstrumentes 16 genutzt. Allerdings hat der Kugelmittelpunkt 26 einen Abstand von der Oberfläche 24 des Körperteils oder Objekts. Dadurch können sich beispielsweise in Körperfurchen Abtastsituationen ergeben, in denen der Kugelmittelpunkt nicht mehr eindeutig einem Punkt auf der Oberfläche des Körpers zugeordnet werden kann.

Um dieses Problem zu umgehen, werden mittels des Abtastinstrumentes 16 erfasste, auf den Kugelmittelpunkt der Abtastspitze 20 als Positionsreferenzpunkt bezogene Positionswerte nicht einem Punkt auf einer Modelloberfläche zugeordnet, wie sie sich aus einem topographischen oder tomographischen Abbild des jeweiligen Körperteils oder Objektes ergibt. Vielmehr wird aus der jeweiligen Modelloberfläche eine virtuelle Modelloberfläche 28 berechnet, die von der Modelloberfläche einen Abstand hat, der genau dem Radius der teilkugelförmigen Abtastspitze entspricht. Diese virtuelle Modelloberfläche 28 ist in Fig. 2 strichpunktiert dargestellt. Die virtuelle Modelloberfläche wird durch die Endpunkte von Flächennormalen auf der Modelloberfläche bestimmt, wobei die Länge der Flächennormalen dem Radius der Abtastspitze entspricht. Die ist durch einige, Flächennormalen entsprechenden Pfeile in Figur 2 angedeutet. Auf den Kugelmittelpunkt der Abtastspitze als Positionsreferenzpunkt bezogene Positionswerte können der virtuellen Modelloberfläche 28 eindeutig und unabhängig von dem Winkel zugeordnet werden, mit dem das Abtastinstrument 16 auf die Oberfläche des Körperteils oder Objektes aufgesetzt wird.

Somit wird es möglich, auch mit einem Abtastinstrument, welches eine Abtastspitze mit im Vergleich zum Stand der Technik großen Radius besitzt, positionsgenau zu erfassen. Eine Abtastspitze mit großem Radius bietet darüber hinaus den Vorteil, dass eine derartige Abtastspitze sich nicht so leicht in Körperfurchen oder Ähnlichem verfängt.

### Bezugszeichenliste

- 10: Kopf
- 12: Feldgenerator
- 14: Referenzpositionssensor
- 16: Abtastinstrument
- 18: Handgriff
- 20: Abtastspitze
- 22: Instrumentenpositionssensor
- 24: Oberfläche
- 26: Kugelmittelpunkt
- 28: Modelloberfläche

## Patentansprüche

1. Verfahren zum Zuordnen von mittels eines Positionserfassungssystems erfasster Positionswerte zu einem topografischen Abbild eines Objektes oder Körperteils, wobei das Verfahren die folgenden Schritte umfasst:
- Bereitstellen eines topografischen Modells eines Objektes oder Körperteils
- Abtasten einer Oberfläche des Objektes oder Körperteils mit einem Abtastinstrument, welches eine Abtastspitze mit einer teilkugelförmigen Außenoberfläche besitz,
- Erfassen von Positionswerten während des Abtastens und
- Zuordnen erfasster Positionswerte zu entsprechenden Positionen in dem topographischen Modell, wobei die erfassten Positionswerte Punkten einer virtuellen Oberfläche des topographischen Modells zugeordnet werden, die von einer Modelloberfläche einen Abstand hat, der gleich einem Radius einer teilkugelförmigen Abtastspitze eines Abtastinstrumentes ist, mit dem die Oberfläche des Objekts oder Körperteils jeweils abgetastet wird.

2. Verfahren gemäß Anspruch 1, **gekennzeichnet durch** den Verfahrensschritt
Erstellen einer Transformationsvorschrift zum Transformieren von Positionswerten und/oder Koordinaten des Positionserfassungssystems zu Modellpositionswerten und/oder Modellkoordinaten des topografischen Modells oder umgekehrt.

3. Verfahren gemäß Anspruch 1 oder 2, **dadurch gekennzeichnet, dass** als Positionserfassungssystem ein Positionserfassungssystem mit einem Feldgenerator für ein elektromagnetisches Wechselfeld und mit wenigstens zwei Positionssensoren mit jeweils wenigstens einer elektrischen Spule verwendet wird, von denen ein Positionssensor so mit dem Objekt oder Körperteil verbunden wird, dass dieser Positionssensor eine feste Relativposition bezüglich des Objektes oder Körperteils beibehält während der andere Positionssensor an dem Abtastinstrument befestigt ist.

4. Verfahren gemäß einem der Ansprüche 1 bis 3, **dadurch gekennzeichnet, dass** das topografische Modell mittels eines tomografischen Verfahrens aufgenommen wird.

5. Verfahren gemäß einem der Ansprüche 1 bis 4, **dadurch gekennzeichnet, dass** beim Zuordnen der erfassten Positionswerte zu entsprechenden Positionen in dem topografischen Modell mehrere an unterschiedlichen Positionen des Objektes oder Körperteils aufgenommene Positionswerte derart Punkten auf der virtuellen Modelloberfläche zugeordnet werden, dass sich bei linearer Skalierung und Rotation ein möglichst kleiner Fehler ergibt.

6. Positionserfassungssystem mit einem beweglichen Abtastinstrument (16), wobei das bewegliche Abtastinstrument (16) eine Abtastspitze (20) mit einer teilkugelförmigen Außenoberfläche aufweist und der Kugelmittelpunkt (26) der sphärischen Abtastspitze (20) als Positionsreferenzpunkt dient und das Positionserfassungssystem weiterhin einen Speicher für ein topographisches Modell eines Objektes oder Körperteils aufweist **dadurch gekennzeichnet, dass** das Positionserfassungssystem dazu ausgebildet ist, von einem beweglichen Instrument erfasste Positionswerte mittels einer Transformationsvorschrift in ein dem topographischen Modell zugrundeliegendes Modellkoordinatensystem derart zu übertragen, dass mittels des Abtastinstruments (16) mit teilkugelförmiger Abtastspitze (20) erfasster Positionswerte zu Punkten oder Positionen auf einer durch das topographische Modell definierten Modelloberfläche (28) zugeordnet werden, indem das Positionserfassungssystem auf den Kugelmittelpunkt (26) der Abtastspitze (20) als Positionsreferenzpunkt bezogene Positionswerte einer virtuellen Oberfläche zuordnet, die von einer durch das topographische Modell definierten Modelloberfläche (28) einen Abstand hat, der genau dem Kugelradius der teilkugelförmigen Abtastspitze (20) entspricht.

7. Positionserfassungssystem gemäß Anspruch 6, **dadurch gekennzeichnet, dass** das Positionserfassungssystem weiterhin dazu ausgebildet ist, zum Erstellen der Transformationsvorschrift ein Zuordnen mittels des Abtastinstruments (16) erfasster Positionswerte zu Punkten auf einer durch das topografische Modell definierten Modelloberfläche (28) durchzuführen, indem das Positionserfassungssystem eine virtuelle Modelloberfläche bestimmt, die von der Modelloberfläche einen Abstand hat, der gleich dem Abstand des Kugelmittelpunktes (26) der Abtastspitze (20) von deren sphärischer Außenoberfläche ist und mittels des Abtastinstruments (16) erfasste Positionswerte unter Beibehalten von deren relativer Position zueinander möglichst genau der virtuellen Modelloberfläche zuordnet.

8. Positionserfassungssystem nach Anspruch 6 oder 7, **dadurch gekennzeichnet, dass** Positionserfassungssystem ein Positionserfassungssystem mit einem Feldgenerator (12) für ein elektromagnetisches Wechselfeld und mit wenigstens zwei Positionssensoren mit jeweils wenigstens einer elektrischen Spule ist, von denen ein Positionssensor (14) so mit dem Objekt oder Körperteil (10) zu verbinden ist, dass dieser Positionssensor (14) eine feste Relativposition bezüglich des Objektes oder Körperteils (10) beibehält während der andere Positionssensor an dem Abtastinstrument befestigt ist.

9. Positionserfassungssystem nach einem der Ansprüche 6 bis 8, **dadurch gekennzeichnet, dass** das Abtastinstrument (16) ein proximales Ende mit einem Handgriff (18) und eine distales Ende mit einer teilkugelförmigen Abtastspitze (20) aufweist, die über einen Schaft mit dem Handgriff (18) verbunden ist, und der Positionssensor des Abtastinstruments ausgebildet ist, Positionswerte zu liefern, die eine Lage und Ausrichtung des Abtastinstruments (16) in Bezug auf einen Referenzsensor (14) liefern, wobei der Positionssensor eine bekannte, feste Lage zum Kugelmittelpunkt (26) der teilkugelförmigen Abtastspitze (20) hat und das Positionserfassungssystem ausgebildet ist, aus Positionswerten des Positionssensors des Abtastinstrumentes (16) Positionswerte des Kugelmittelpunktes (26) der teilkugelförmigen Abtastspitze (20) als Positionsreferenzwerte des Abtastinstrumentes (16) für das Zuordnen von Positionswerten zu Punkten auf einer durch das topografische Modell definierten Modelloberfläche (28) abzuleiten

## Claims

1. Method for assigning position values, detected by means of a position detection system, to a topographic image of an object or body part, the method comprising the following steps:
- providing a topographic model of an object or body part,
- scanning a surface of the object or body part by means of a scanning instrument, which has a scanning tip with a partly spherical external surface,
- detecting position values during the scanning and
- assigning detected position values to corresponding positions in the topographic model, the detected position values being assigned to points of a virtual surface of the topographic model, which has a distance from a model surface which equals a radius of a partly spherical scanning tip of a scanning instrument, by means of which the surface of the object or body part is scanned in each case.

2. Method according to Claim 1, **characterized by** the method step:
establishing a transformation prescription for transforming position values and/or coordinates of the position detection system to model position values and/or model coordinates of the topographic model, or vice versa.

3. Method according to Claim 1 or 2, **characterized in that** a position detection system comprising afield generator for an alternating electromagnetic field and comprising at least two position sensors, each with at least one electrical coil, is used as position detection system, of which position sensors one position sensor is connected to the object or body part in such a way that this position sensor maintains a fixed relative position in relation to the object or body part, while the other position sensor is fastened to the scanning instrument.

4. Method according to one of Claims 1 to 3, **characterized in that** the topographic model is recorded by means of a tomographic method.

5. Method according to one of Claims 1 to 4, **characterized in that**, when assigning the detected position values to corresponding positions in the topographic model, a plurality of position values recorded at different positions of the object or body part are assigned to points on the virtual model surface in such a way that this results in an error which is as small as possible in the case of linear scaling and rotation.

6. Position detection system comprising a movable scanning instrument (16), the movable scanning instrument (16) having a scanning tip (20) with a partly spherical external surface and the sphere center (26) of the sphere-shaped scanning tip(20) serving as position reference point and the position detection system furthermore comprising a storage medium for a topographic model of an object or body part **characterized in that** the position detection system is embodied to transfer position values detected by a movable instrument into a model coordinate system, underlying the topographic model, by means of a transformation prescription in such a way that position values detected by means of the scanning instrument (16) with partly spherical scanning tip(20) are assigned to points or positions on a model surface (28)defined by the topographic model by virtue of the position detection system assigning position values, related to the sphere center (26) of the scanning tip (20) as position reference point, to a virtual surface which has a distance from a model surface (28) defined by the topographic model, which distance corresponds precisely to the sphere radius of the partly spherical scanning tip (20).

7. Position detection system according to Claim 6, **characterized in that** the position detection system is furthermore embodied, for establishing the transformation prescription, to carry out an assignment of position values detected by means of the scanning instrument (16) to points on a model surface (28) defined by the topographic model by virtue of the position detection system determining a virtual model surface which has a distance from the model surface (28) which equals the distance of the sphere center (26) of the scanning tip (20) from the sphere-shaped external surface thereof and assigning position values detected by means of the scanning instrument (16) as precisely as possible to the virtual model surface while maintaining the relative position of said position values to one another.

8. Position detection system according to Claim 6 or 7, **characterized in that** the position detection system is a position detection system comprising a field generator (12) for an alternating electromagnetic field and comprising at least two position sensors, each with at least one electrical coil, of which position sensors (14) one position sensor is to be connected to the object or body part (10) in such a way that this position sensor (14) maintains a fixed relative position in relation to the object or body part (10), while the other position sensor is fastened to the scanning instrument.

9. Position detection system according to one of Claims 6 to 8, **characterized in that** the scanning instrument (16) has a proximal end with a handle (18) and a distal end with a partly spherical scanning tip (20), which is connected to the handle (18) via a shank, and the position sensor of the scanning instrument (16) is embodied to supply position values which supply a location and alignment of the scanning instrument in relation to a reference sensor (14), the position sensor having a known, fixed location from the sphere center (26) of the partly spherical scanning tip (20) and the position detection system being embodied to derive, from position values of the position sensor of the scanning instrument (16), position values of the sphere center (26) of the partly spherical scanning tip (20) as position reference values of the scanning instrument (16) for assigning position values to points on a model surface (28)defined by the topographic model.

## Revendications

1. Dispositif (10) électronique de production automatique d'une liste (18) triée d'articles se rapportant à un article germe,
le dispositif (10) électronique comprenant un déterminateur (20) de parenté, configuré pour comparer l'article germe à une pluralité d'autres articles et pour déterminer ainsi une valeur de parenté avec l'article germe pour chaque autre article,
le dispositif (10) comprenant, en outre, un moteur (24) de groupage, configuré pour grouper les autres articles en déterminant une parenté relative parmi les autres articles, chaque autre article étant ainsi affecté à un groupe, et
le dispositif (10) comprenant, en outre, un producteur (28) de liste, configuré pour produire la liste (18) triée, le producteur (28) de liste comprenant un créditeur (30) de groupe et un sélectionneur (32) d'article,
dans lequel le créditeur (30) de groupe est configuré pour affecter initialement des crédits initiaux aux groupes, les crédits initiaux ayant une valeur dépendant de la valeur de parenté de l'article ayant la valeur de parenté la plus grande dans chacun des groupes et
dans lequel le créditeur (30) de groupe est configuré pour, en d'autres stades itératifs :
mettre à jour les crédits en ajoutant un crédit ajouté aux groupes, tout en ne modifiant pas la valeur de parenté des articles des groupes, et
dans lequel le sélectionneur (32) d'article est configuré pour, dans chacun des autres stades itératifs :
sélectionner un article, qui doit être ajouté à la liste (18) triée, qui a la valeur de parenté la plus grande dans un groupe ayant le crédit le plus grand, pour ajouter l'article sélectionné à la liste (18) triée et pour retirer l'article sélectionné du groupe auquel il appartient, et
dans lequel le créditeur (30) de groupe est configuré pour, dans chacun des autres stades itératifs :
diminuer le crédit du groupe, dont l'article sélectionné, qui a été ajouté à la liste (18) triée, a été retiré.

2. Dispositif (10) électronique de production automatique d'une liste (18) triée d'articles en relation avec un article germe,
le dispositif (10) électronique comprenant un déterminateur (20) de parenté, configuré pour comparer l'article germe à une pluralité d'autres articles et pour déterminer ainsi une valeur de parenté avec l'article germe pour chaque autre article,
le dispositif (10) comprenant, en outre, un moteur (24) de groupage, configuré pour grouper les autres articles en déterminant une parenté relative parmi les autres articles, chaque autre article étant ainsi affecté à un groupe, et
le dispositif (10) comprenant, en outre, un producteur (28) de liste, configuré pour produire la liste (18) triée, le producteur (28) de liste comprenant un créditeur (30) de groupe et un sélectionneur (32) d'article,
dans lequel le créditeur (30) de groupe est configuré pour affecter initialement des crédits initiaux aux groupes, les crédits initiaux ayant une valeur déterminée à l'avance, et
dans lequel le créditeur (30) de groupe est figuré pour, dans d'autres stades itératifs :
mettre à jour les crédits en ajoutant un crédit ajouté aux groupes, le crédit ajouté dépendant de la valeur de parenté de l'article ayant la valeur de parenté la plus grande dans l'un respectif des groupes, en relation avec la valeur de parenté de l'article ayant la valeur de parenté la plus grande dans les autres groupes, tout en ne modifiant pas la valeur de parenté des articles des groupes, et
dans lequel le sélectionneur (32) d'article est configuré pour, dans chacun des autres stades itératifs :
sélectionner un article, qui doit être ajouté à la liste (18) triée, qui a la valeur de parenté la plus grande dans un groupe ayant le crédit le plus grand, pour ajouter l'article sélectionné à la liste (18) triée et pour retirer l'article sélectionné du groupe auquel il appartient, et
dans lequel le créditeur (30) de groupe est configuré pour, dans chacun des autres stades itératifs :
diminuer le crédit du groupe, dont l'article sélectionné, qui a été ajouté à la liste (18) triée, a été retiré.

3. Dispositif (10) électronique suivant la revendication 1 ou la revendication 2, dans lequel le créditeur (30) de groupe est configuré pour mettre à jour des crédits seulement pour les groupes, qui comprennent au moins un article ayant une valeur de parenté, qui dépasse une fraction déterminée à l'avance de la valeur de parenté la plus grande de tous les articles, qui n'ont pas encore été ajoutés à la liste et retirés des groupes.

4. Dispositif (10) électronique suivant la revendication 3, dans lequel il est prévu une interface d'utilisateur de réglage du seuil, qui permet d'entrer une valeur de seuil, qui détermine la fraction.

5. Dispositif (10) électronique suivant l'une des revendications 1 à 4, comprenant, en outre, un producteur de demande pour produire des demandes à envoyer à des dépôts d'articles et reposant sur un article germe, et pour recevoir des résultat de ces demandes représentant des articles à trier.

6. Procédé de tri d'articles en créant une liste triée, le procédé comprenant les stades dans lesquels :
- on se procure un article germe,
- on compare une pluralité d'autres articles à l'article germe et on détermine une valeur de parenté pour chaque autre article par rapport à l'article germe,
- on groupe les autres articles en un certain nombre de groupes, en comparant les autres articles les uns avec les autres, pour déterminer une parenté relative entre les autres article, et on groupe les autres articles en groupes, de manière à ce que chaque article appartienne à un seul groupe, qui contient des articles ayant une parenté relativement grande entre eux par rapport à des articles d'autres groupes,
- on produit une liste (18) triée à partir des autres articles, en organisant les autres articles en fonction de leur valeur de parenté et de leur appartenance à un certain groupe d'une manière itérative, des articles étant ajoutés à la liste un par un et initialement
a) en affectant des crédit initiaux aux groupes, les crédits initiaux ayant une valeur, qui dépend de la valeur de parenté de l'article ayant la valeur de parenté la plus grande dans chacun des groupes,
et dans chaque stade itératif,
b) en mettant à jour des crédits en ajoutant un crédit ajouté aux groupes, tout en ne modifiant pas la valeur de parenté des articles des groupes,
c) en sélectionnant un article, qui doit être ajouté à la liste (18) triée, qui a la valeur de parenté la plus grande dans un groupe ayant le crédit le plus grand,
d) en ajoutant l'article sélectionné à la liste (18) triée et en retirant l'article sélectionné du groupe, et
e) en diminuant le crédit du groupe, dont l'article ajouté a été retiré,
en répétant les stades b) à e) jusqu'à ce que tous les autres articles, ou jusqu'à ce qu'un nombre déterminé à l'avance des autres articles ait été ajouté à la liste (18) triée.

7. Procédé de tri d'articles en créant une liste triée, le procédé comprenant les stades dans lesquels :
- on se procure un article germe,
- on compare une pluralité d'autres articles à l'article germe et on détermine une valeur de parenté pour chaque autre article par rapport à l'article germe,
- on groupe les autres articles en un certain nombre de groupes, en comparant les autres articles les uns avec les autres, pour déterminer une parenté relative entre les autres articles, et on groupe les autres articles en groupes, de manière à ce que chaque article appartienne à un seul groupe, qui contient des articles ayant une parenté relativement grande entre eux par rapport à des articles d'autres groupes,
- on produit une liste (18) triée à partir des autres articles, en organisant les autres articles en fonction de leur valeur de parenté et de leur appartenance à un certain groupe d'une manière itérative, des articles étant ajoutés à la liste un par un et initialement
a) en affectant des crédits initiaux ayant une valeur déterminée à l'avance,
et dans chaque stade itératif,
b) en mettant à jour des crédits, en ajoutant un crédit ajouté aux groupes, le crédit ajouté dépendant de la valeur de parenté de l'article ayant la valeur de parenté la plus grande dans l'un respectif des groupes, en relation à la valeur de parenté de l'article ayant la valeur de parenté la plus grande dans les autres groupes, tout en ne modifiant pas la valeur de parenté des articles des groupes,
c) en sélectionnant un article, qui doit être ajouté à la liste (18) triée, qui a la valeur de parenté la plus grande dans un groupe ayant le crédit le plus grand,
d) en ajoutant l'article sélectionné à la liste (18) triée et en retirant l'article sélectionné du groupe, et
e) en diminuant le crédit du groupe, dont l'article ajouté a été retiré,
en répétant les stades b) à e) jusqu'à ce que tous les autres article, ou jusqu'à ce qu'un nombre déterminé à l'avance des autres articles ait été ajouté à la liste (18) triée.

8. Procédé suivant la revendication 6 ou 7, dans lequel on effectue le stade b) « mise à jour des crédits » seulement sur les groupes comprenant des articles, qui ont une valeur de parenté plus grande qu'une fraction déterminée à l'avance de la valeur de parenté la plus grande de tous les autres articles de n'importe quel groupe.

9. Procédé suivant l'une des revendications 6 à 8, dans lequel le stade de groupage des autres articles comprend
- commencer avec *N* groupes, chaque groupe contenant un article singulier unique
- fusionner deux groupes les plus semblables, la similitude entre deux groupes étant définie par la parenté relative minimum ou moyenne entre leurs groupes, et
- répéter la fusion de groupe jusqu'à obtention d'un nombre déterminé à l'avance de groupes.
